(19) Europäisches Patentamt
European Patent Office
Office européen des brevets

(11) Veröffentlichungsnummer: **0 165 472**
**B1**

(12) **EUROPÄISCHE PATENTSCHRIFT**

(45) Veröffentlichungstag der Patentschrift: **06.02.91**

(21) Anmeldenummer: **85106030.1**

(22) Anmeldetag: **15.05.85**

(51) Int. Cl.⁵: **A 61 B 10/00,** A 61 B 17/28, A 61 B 17/32

(54) Chirurgisches Greiferinstrument.

(30) Priorität: **18.05.84 DE 8415222 u**

(43) Veröffentlichungstag der Anmeldung:
**27.12.85 Patentblatt 85/52**

(45) Bekanntmachung des Hinweises auf die Patenterteilung:
**06.02.91 Patentblatt 91/06**

(84) Benannte Vertragsstaaten:
**AT DE FR GB NL**

(56) Entgegenhaltungen:
**DE-A-2 817 922**
**DE-B-2 506 471**
**DE-B-2 558 570**
**US-A-2 113 246**

(73) Patentinhaber: **Maslanka, Harald**
**Stockacher Strasse 172**
**D-7200 Tuttlingen (DE)**

(72) Erfinder: **Maslanka, Harald**
**Stockacher Strasse 172**
**D-7200 Tuttlingen (DE)**

(74) Vertreter: **Weickmann, Heinrich, Dipl.-Ing. et al**
**Patentanwälte Dipl.-Ing. H.Weickmann Dipl.-Phys.Dr. K.Fincke Dipl.-Ing. F.A.Weickmann Dipl.-Chem. B. Huber Dr.-Ing. H. Liska Dipl.-Phys.Dr. J. Prechtel Postfach 860820 D-8000 München 86 (DE)**

Courier Press, Leamington Spa, England.

## Beschreibung

Die Erfindung betrifft ein chirurgisches Greiferinstrument, insbesondere eine Probenexzessions-Zange, wie sie beispielsweise bei der Broncho-, Bulbo-, Kolo-, Duodeno-, Endo- oder Gastroskopie eingesetzt werden kann.

Ein zum Beispiel aus DE-A-2 506 471 bekanntes Greiferinstrument dieser Art umfaßt einen Greifer sowie ein langgestrecktes, an seinem patientennahen Ende den Greifer haltendes, flexibles Betätigungskabel, welches einen radial dichten Kabelmantel und eine in dem Kabelmantel verschiebbar geführte, bei der Relativverschiebung den Greifer betätigende Kabelseele aufweist. Am patientenfernen Ende des Betätigungskabels ist eine Handbetätigungseinrichtung angeschlossen. Die Handbetätigungseinrichtung umfaßt einen langgestreckten Führungsschaft, der an seinem einen Ende ein in Schaftrichtung sich erstreckendes Befestigungsrohr, an den der Kabelmantel koaxial befestigt ist, und an seinem anderen Ende einen ersten Fingergriff in Form eines Daumenrings trägt. An dem Führungsschaft ist ein zweiter Fingergriff in Form eines Mittelfinger-Zeigefinger-Schiebegriffs verschiebbar geführt, der über eine das Befestigungsrohr durchsetzende, starre Kupplungsstange mit der flexiblen Kabelseele verbunden ist.

Bei derartigen, bekannten Greiferinstrumenten bilden der Greifer, das Betätigungskabel und die Handbetätigungseinrichtung eine betriebsmäßig untrennbare Einheit. Dies hat zwar den Vorteil, daß das an sich empfindliche Greiferinstrument stabiler und mit geringerem konstruktiven Aufwand gebaut werden kann, hat aber andererseits den Nachteil, daß es nur sehr schwer zu reinigen ist. Insbesondere kann bei Benutzung das Innere des Betätigungskabels verschmutzen, mit der Folge, daß die empfindliche, über eine Hebelschere angetriebene Zange blockiert und das Instrument unbrauchbar wird.

Aufgabe der Erfindung ist es, ein chirurgisches Greiferinstrument, bei welchem der Greifer, das Betätigungskabel und die Handbetätigungseinrichtung eine betriebsmäßig nicht zerlegbare Einheit bilden, so zu verbessern, daß es leicht und vollständig gereinigt werden kann.

Diese Aufgabe wird erfindungsgemäß dadurch gelöst, daß der Kabelmantel abgedichtet an dem Befestigungsrohr angebracht ist, daß das Befestigungsrohr einen Spülanschlußstutzen trägt, der in einem zwischen dem Befestigungsrohr und der Kupplungsstange gebildeten, bis in das Innere des Kabelmantels hineinreichenden Ringraum mündet und daß das Befestigungsrohr auf der dem Betätigungskabel abgewandten Seite des Spülanschlußstutzens eine die Kupplungsstange umschließende und zum Befestigungsrohr hin abdichtende Ringdichtung trägt. Über den Spülanschlußstutzen kann das Innere des Kabelmantels nach Benutzung des Instruments mit Spül- und Desinfektionsflüssigkeit zum Greifer hin gespült werden, ohne daß das Instrument für eine vollständige Reinigung zerlegbar sein muß.

In einer bevorzugten Ausführungsform ist der Kabelmantel an einer, die Kupplungsstange umschließenden Hülse koaxial befestigt, wobei die Hülse in das Befestigungsrohr eingreift und ihrerseits an diesem befestigt ist. Der mit dem Kabelmantel verbundene Ringraum befindet sich hierbei zwischen der Hülse und der Kupplungsstange. Für die Verbindung zum Spülanschluß ist im Bereich des Spülanschlusses ein zweiter Ringraum zwischen dem Befestigungsrohr und der Hülse vorgesehen, der über wenigstens eine radiale Öffnung der Hülse mit dem erstgenannten Ringraum verbunden ist. Die Ringdichtung ist zweckmäßigerweise zwischen der patientenfernen axialen Stirnseite der Hülse und einer zur Hülse weisenden, radial nach innen vorspringenden Schulterfläche des Befestigungsrohrs eingespannt. Für die Abdichtung der Hülse zum Befestigungsrohr hin hat es sich als günstig erwiesen, zwischen der patientennahen, axialen Stirnfläche des Befestigungsrohrs und einer axial zum Befestigungsrohr weisenden, radial nach außen vorspringenden Ringschulter der Hülse einen weiteren Dichtring einzuspannen. Die axialen Einspannkräfte werden mittels einer auf das Befestigungsrohr geschraubten Überwurfmutter erzeugt, die die Hülse gegen die Dichtringe spannt und an dem Befestigungsrohr hält.

Im folgenden soll ein Ausführungsbeispiel der Erfindung anhand von Zeichnungen näher erläutert werden.

Es zeigt:

Figur 1 eine Probenexzessions-Zange mit in Draufsicht dargestellter Handbetätigungseinrichtung und in vergrößertem Schnitt dargestellter Zange und

Figur 2 eine vergrößerte Darstellung des patientennahen Endes der Handbetätigungseinrichtung.

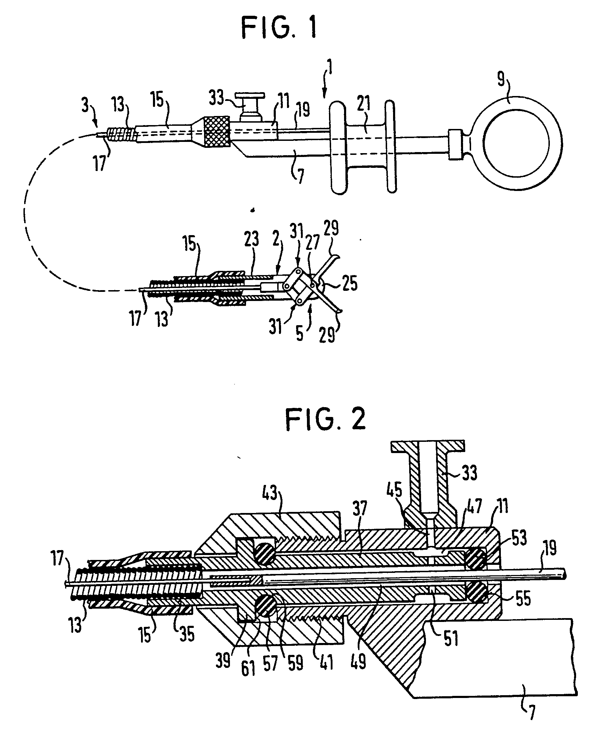

Die in den Figuren dargestellte, chirugische Probenexzessions-Zange umfaßt eine Handbetätigungseinrichtung 1, die über ein flexibles, vergleichweise dünnes, aber langes Betätigungskabel 3 mit einer Zange 5 zu einer betriebsmäßig nicht trennbaren Einheit verbunden ist. Die Handbetätigungseinrichtung 1 umfaßt einen Führungsschaft 7, der an seinem patientenfernen Ende einen Daumenring 9 trägt. Am patientennahen Ende des Führungsschafts 7 ist ein Befestigungsrohr 11 angebracht, an dem in nachstehend noch näher erläuterter Weise ein durch eine Federspirale gebildeter Kabelmantel 13 des Betätigungskabels 3 befestigt ist. In dem Kabelmantel 13, der durch eine Kunststoffummantelung 15 nach außen hin abgedichtet ist, ist eine flexible Kabelseele 17 verschiebbar geführt. Die Kabelseele 17 ist über eine das Befestigungsrohr 11 durchsetzende Kupplungsstange 19 mit einem auf dem Führungsschaft 7 verschiebbar geführten Mittelfinger-Zeigefinger-Schiebegriff 21 verbunden. Die Zange 5 ist am patientennahen Ende des Betätigungskabels 3 gehalten und umfaßt ein koaxial am Kabelmantel 13 befestigtes Gabelrohr 23, welches auf seiner kabelmantelfernen Seite 2 einander diametral gegenüberliegende Gabelhälften 25 trägt. An den Gabelhälften 25, von denen in Fig. 1 lediglich eine dargestellt ist, sind an einer diametralen Achse 27 zwei Zangenhälften 29 relativ zueinander und zum Gabelrohr 23 schwenkbar gelagert. Die Zangenhälften 29 sind über Scherenhebel 31 gelenkig mit der Kabelseele 17 gekuppelt. Werden der Daumengriff 9 und der Zeigefinger-Mittelfinger-Schiebegriff 21 aufeinander zubewegt, so wird die Zange 5 geschlossen. Wird der Daumengriff 9 und der Zeigefinger-Mittelfinger-Schiebegriff 21 voneinander wegbewegt, so die Zange 5 geöffnet.

Die Probenexzessions-Zange kann betriebsmäßig nicht zerlegt werden. Um das Betätigungskabel 3 und insbesondere auch die empfindliche Zange 5 reinigen und desinfizieren zu können, ist an dem Befestigungsrohr 11 ein Spülanschlußstutzen 33, insbesondere ein Luer-Lock-Anschluß befestigt. Der Spülanschlußstutzen 33 ist, wie am besten Fig. 2 zeigt, mit dem Innenraum des Kabelmantels 13 verbunden. Der Kabelmantel 13 ist in einer stirnseitigen Aussparung 35 einer die Kupplungsstange 19 koaxial umschließenden und koaxial in dem Befestigungsrohr 11 sitzenden Hülse 37 gehalten. Auf der patientennahen Seite des Befestigungsrohrs 11 trägt die Hülse 37 einen radial nach außen vorspringenden Ringansatz 39, über den eine auf ein Außengewinde 41 des Befestigungsrohrs 11 geschraubte Überwurfmutter 43 die Hülse 37 an dem Befestigungsrohr 11 hält. Die Ummantelung 15 überdeckt auch den auf der patientennahen Seite der Überwurfmutter 43 vorstehenden Bereich der Hülse 37.

Im Bereich des Spülanschlußstutzens 33, der durch eine radiale Öffnung 45 mit dem Innenraum des Befestigungsrohrs 11 verbunden ist, ist die Hülse 37 mit einer über ihren Außenumfang umlaufenden Ringnut 47 versehen. Die Ringnut 47 bildet einen Ringraum, über den sich die Spülflüssigkeit am Umfang der Hülse 37 verteilen kann. Zwischen dem Innenmantel der Hülse 37 und der Kupplungsstange 19 ist ein weiterer Ringraum 49 gebildet, der über mehrere radiale Öffnungen 51 am Boden der Ringnut 47 mit dieser verbunden ist. Der Ringraum 49 erstreckt sich bis in den Innenraum des Kabelmantels 13 hinein.

Zur patientenfernen Seite hin ist der Ringraum 49 durch eine die Kupplungsstange 19 dicht umschließende Ringdichtung 53 abgedichtet, die zwischen der patientenfernen axialen Stirnseite der Hülse 37 und einer radial nach innen vorspringenden, axial zur Hülse 37 weisenden Ringschulter 55 des Befestigungsrohrs 11 eingespannt ist. Eine weitere Ringdichtung 57, die in eine den Außenumfang der Hülse 37 umschließende Ringnut 59 eingeschnappt ist, dichtet die patientennahe, axiale Stirnfläche des Befestigungsrohrs 11 gegen die durch den Ringansatz 39 gebildete, zum Befestigungsrohr 11 axial gerichtete Ringschulter 61 ab. Die Spannkräfte werden mittels der Überwurfmutter 43 erzeugt.

Durch Einspülen einer Spülflüssigkeit in den Spülanschlußstutzen 33 kann das Innere des Kabelmantels 13 einschließlich der ansonsten nur schwer zugänglichen Gelenkteile der Zange 5 gereinigt und desinfiziert werden.

## Patentansprüche

1. Chirurgisches Greiferinstrument, insbesondere Probenexzessions-Zange, umfassend einen Greifer (5), ein langgestrecktes, an seinem patientennahen Ende den Greifer (5) haltendes, flexibles Betätigungskabel (3), welches einen radial dichten Kabelmantel (13) und eine in dem Kabelmantel (13) verschiebbar geführte, bei der Relativverschiebung den Greifer (5) betätigende Kabelseele (17) aufweist und eine am patientenfernen Ende des Betätigungskabels (3) angeschlossene Handbetätigungseinrichtung (1) mit einem langgestreckten Führungsschaft (7), der an seinem einen Ende ein in Schaftrichtung sich erstreckendes Befestigungsrohr (11), an dem der Kabelmantel (13) koaxial befestigt ist, und an seinem anderen Ende einen ersten Fingergriff (9) trägt und mit einem an dem Führungsschaft (7) verschiebbar geführten zweiten Fingergriff (21), der über eine das Befestigungsrohr (11) durchsetzende Kupplungsstange (19) mit der Kabelseele (17) verbunden ist, dadurch gekennzeichnet, daß der Kabelmantel (13) abgedichtet an dem Befestigungsrohr (11) angebracht ist, daß das Befestigungsrohr (11) einen Spülanschlußstutzen (33) trägt, der in einen zwischen dem Befestigungsrohr (11) und der Kupplungsstange (19) gebildeten, bis in das Innere des Kabelmantels (13) hineinreichenden Ringraum (49) mündet und daß das Befestigungsrohr (11) auf der dem Betätigungskabel (3) abgewandten Seite des Spülanschlußstutzens (33) eine die Kupplungsstange (19) umschließende und zum Befestigungsrohr (11) hin abdichtende Ringdichtung (53) trägt.

2. Chirurgisches Greiferinstrument nach Anspruch 1, wobei der Kabelmantel (13) an einer die Kupplungsstange (19) umschließenden Hülse (37) koaxial befestigt ist und die Hülse (37) in das Befestigungsrohr (11) eingreift und ihrerseits an diesem befestigt ist, dadurch gekennzeichnet, daß der bis in den Kabelmantel (13) reichende (erste) Ringraum (49) zwischen der Hülse (37) und der Kupplungsstange (19) vorgesehen ist und daß zumindest im Bereich des Spülanschlußstutzens (33) ein zweiter Ringraum (47) gebildet ist, der über wenigstens eine radiale Öffnung (51) der Hülse (37) mit dem ersten Ringraum (49) verbunden ist.

3. Chirurgisches Greiferinstrument nach Anspruch 2, dadurch gekennzeichnet, daß der zweite Ringraum (47) durch eine Außendurchmesserverkleinerung der Hülse (37) gebildet ist.

4. Chirurgisches Greiferinstrument nach Anspruch 3 oder 4, dadurch gekennzeichnet, daß das Befestigungsrohr (11) auf der patientenfernen Seite der Hülse (37) eine radial nach innen vorspringende, axial zur Hülse (37) weisende Ringschulter (55) aufweist und daß der Dichtring (53) axial zwischen der Ringschulter (55) und der patientenfernen, axialen Stirnfläche der Hülse (37) angeordnet ist.

5. Chirurgisches Greiferinstrument nach einem der Ansprüche 2 bis 4, wobei die Hülse (37) auf der patientennahen Seite des Befestigungsrohrs (11) eine radial nach außen vorspringende, axial zum Befestigungsrohr weisende Ringschulter (61) aufweist und mittels einer auf das patientennahe Ende des Befestigungsrohrs (11) geschraubten Überwurfmutter (43) an dem Befestigungsrohr (11) gehalten ist, dadurch gekennzeichnet, daß axial zwischen der Ringschulter (61) und der patientennahen, axialen Stirnfläche des Befestigungsrohrs (11) ein die Hülse (37) umschließender Dichtring (57) angeordnet ist.

## Revendications

1. Instrument chirurgical à pince, notamment pince pour prélèvements biopsiques, comportant une pince (5), un câble d'actionnement (3) flexible, allongé, maintenant la pince (5) à son extrémité proche du patient et qui présente une gaine de câble (13) radialement étanche et une âme de câble (17) guidée coulissante dans la gaine de câble (13) et actionnant la pince (5), lors de son déplacement relatif, comportant aussi un dispositif d'actionnement manuel (1), raccordé à l'extrémité du câble d'actionnement (3) éloignée du patient, qui porte, à l'une de ses extrémités, un tube de fixation (11) s'étendant dans la direction de la tige et sur lequel est fixée coaxialement la gaine de câble (13) et qui porte à son autre extrémité une première tirette (9) et comporte une deuxième tirette (21), guidée coulissante sur la tige de guidage (2) laquelle est reliée à l'âme de câble (17), par une tringle d'accouplement (19) traversant le tube de fixation (11), caractérisé en ce que la gaine de câble (13) est placée de manière étanche sur le tube de fixation, en ce que le tube de fixation (11) porte une tubulure de lavage (33) qui débouche dans un volume annulaire (49) formé entre le tube de fixation (11) et la tringle d'accouplement (19), s'étendant jusqu'à l'intérieur de la gaine de câble (13) et en ce que le tube de fixation (11) porte sur le côté de la tubulure de lavage (33), opposé au câble d'actionnement (3), une bague d'étanchéité (53) entourant la tringle d'accouplement (19) et assurant l'étanchéité vers le tube de fixation (11).

2. Instrument chirurgical à pince selon la revendication 1, dans lequel la gaine de câble (13) est fixée coaxialement sur un manchon (37) entourant la tringle d'accouplement (19) et le manchon (37) s'engage dans le tube de fixation (11) et est fixé à son tour sur celui-ci, caractérisé en ce que le (premier) espace annulaire (49) s'étendant jusque dans la gaine de câble (13) est prévu entre le manchon (37) et la tringle d'accouplement (39) et en ce qu'un deuxième espace annulaire (47) est formé au moins dans la région de la tubulure de lavage (33) et communique avec le premier espace annulaire (49), par au moins une ouverture radiale (51) du manchon (37).

3. Instrument chirurgical à pince selon la revendication 2, caractérisé en ce que le deuxième espace annulaire (47) est formé par une réduction du diamètre extérieur du manchon (37).

4. Instrument chirurgical à pince selon la revendication 3 ou 4, caractérisé en ce que le tube de fixation (11) présente, sur le côté du manchon (37), éloigné du patient, un épaulement annulaire (55) faisant saillie radialement vers l'intérieur, tourné axialement vers le manchon (37) et en ce que la bague d'étanchéité (53) est disposée axialement, entre l'épaulement annulaire (55) et la face frontale axiale du manchon (37), éloignée du patient.

5. Instrument chirurgical à pince selon l'une des revendications 2 à 4, dans lequel le manchon (37) présente, sur le côté du tube de fixation (11), proche du patient, un épaulement annulaire (61) faisant saillie radialement vers l'extérieur, tourné axialement vers le tube de fixation et est maintenu sur le tube de fixation (11) au moyen d'un écrou (43) vissé sur l'extrémité du tube de fixation (11), proche du patient, caractérisé en ce qu'une bague d'étanchéité (57), entourant le manchon (37), est disposée axialement entre l'épaulement annulaire (61) et la face frontale axiale du tube de fixation (11), proche du patient.

## Claims

1. A surgical gripping instrument, particularly a specimen excession forceps, comprising a gripper (5), an elongated flexible actuating cable (3) supporting the gripper (5) at its end which is near the patient and which comprises a radially sealing-tight cable sheath (13) and a cable core (17) guided for displacement in the cable sheath (13) and which, upon relative displacement, actuates the gripper (5), and comprising, connected to the end of the actuating cable (3) which is remote from the patient, a manual actuating means (1) with an elongated guide stem (7) which has at one end and extending in the direction of the stem a fixing tube (11) on which the cable sheath (13) is coaxially fixed, and which has at its other end a first finger grip (9) and with, guided for displacement on the guide stem (7) a second finger grip (21) which is connected to the cable core (17) via a coupling rod (19) which passes through the fixing tube (11), characterised in that the cable sheath (13) is mounted in sealing-tight fashion on the fixing tube (11) and in that the fixing tube (11) carries a flushing connector (33) which opens out into an annular space (49) formed between the fixing tube (11) and the coupling rod (19) and extending into the interior of the cable sheath (13) and in that the fixing tube (11), on the side of the flushing connector (33) which is remote from the actuating cable (3) has enclosing the coupling rod (19) an annular seal (53) for sealing-tightness in respect of the fixing tube (11).

2. A surgical gripping instrument according to Claim 1, in which the cable sheath (13) is coaxially fixed on a sleeve (37) which encloses the coupling rod (19), the sleeve (37) engaging into and being attached to the fixing tube (11), characterised in that the (first) annular space (49) which extends into the cable sheath (13) is provided between the sleeve (37) and the coupling rod (19) and in that at least in the region of the flushing connector (33) there is a second annular space (47) which is connected to the first annular space (49) via at least one radial aperture (51) in the sleeve (37).

3. A surgical gripping instrument according to Claim 2, characterised in that the second annular space (47) is formed by a reduction in the outside diameter of the sleeve (37).

4. A surgical gripping instrument according to Claim 3 or 4, characterised in that the fixing tube (11) has at the end of the sleeve (37) which is remote from the patient a radially inwardly projecting annular shoulder (55) which is directed axially in relation to the sleeve (37) and in that the packing ring (53) is disposed axially between the annular shoulder (55) and the axial end face of the sleeve (37) which is remote from the patient.

5. A surgical gripping instrument according to one of Claims 2 to 4, in which the sleeve (37) has, at the end of the fixing tube (11) which is close to the patient a radially outwardly projecting annular shoulder (61) which is directed axially in relation to the fixing tube and which is supported on the fixing tube (11) by means of a cap nut (43) screwed onto the end of the fixing tube (11) which is close to the patient, characterised in that axially between the annular shoulder (61) and the axial end face of the fixing tube (11) which is close to the patient there is a packing ring (57) which surrounds the sleeve (37).

# FIG. 1

# FIG. 2